# EUROPEAN PATENT APPLICATION

(11) **EP 3 748 553 A1**
(43) Date of publication of application: **09.12.2020**
(21) Application number: 19305734.6
(22) Date of filing: 06.06.2019
(51) Int. Cl.: G06N 7/00, G06N 3/00, G06J 1/00, G16Z 99/00, C40B 50/00, G16C 20/00

(54) **METHOD FOR DETERMINING AN OPTIMAL DRUG DOSING REGIMEN**

(71) Applicant: ASSISTANCE PUBLIQUE, HOPITAUX DE PARIS, 75004 Paris (FR); UNIVERSITE PARIS DESCARTES, 75006 Paris (FR)
(72) Inventor: HIRT, Déborah, 75014 PARIS (FR); BENABOUD, Nedjma, Sihem, 92120 MONTROUGE (FR); BOUAZZA, Naïm, 75013 PARIS (FR); TRELUYER, Jean-Marc, 75013 PARIS (FR)
(74) Representative: Gevers & Orès

(57) **Abstract**

The invention concerns a method determining an optimum drug dosing regimen to treat a patient efficiently against drug-sensitive pathogenic agents. From relevant patient-related data and the drug concentration measured at a random time in the patient's body, the method estimates the drug concentration time-course based on a Bayesian model. The residual concentration (Cᵣ) and an efficiency pharmacokinetic parameter (PPE) adapted to said drug and patient are computed from the estimated concentration. A dosing regimen is then determined by comparing the PPE to an efficiency target (CE) for efficiency purposes. The method further may further take into account toxicity constraints by comparing the residual drug concentration (Cᵣ) to at least a toxic concentration (Cₜₒₓ). A concentration of said drug is determined (E13) based on the result the above comparisons to provide a proposed dose for said optimum dosing regimen.

## Description

### 1. Field of the invention

The present invention concerns a method for determining drug dosing regimens, in particular in case the drug is an anti-infective drug.

The invention is intended to assist a physician or a doctor in interpreting measured concentrations of a drug and in deciding on most appropriate doses of the drug to administer to a patient to be treated, while taking into account pharmacokinetic considerations and *concentration* - *effects* relationships.

The invention is particularly well-suited to provide an optimal drug dosing regimen that can be adapted individually to each patient, taking into account the characteristics of the patient, the characteristics of the drug used, the characteristics of the pathogenic agent (*e.g.* germ, bacteria or virus) found or suspected in the patient's body and the time at which the drug concentration was measured in the patient's body.

The method according to the present invention contributes to optimize the treatment of a patient by determining the most appropriate dosing regimen (e.g. dose, dosing intervals and the infusion duration) that is adapted to the patient.

The invention may apply to any type of drugs or medicines that need to be administered to a patient, such as anti-infective drugs to combat bacterial, fungal or viral infections.

### 2. Prior art

Even though the interpretation of biological tests may be facilitated by existing standards, the interpretation of drug dosing still remains cumbersome, as it generally depends on the time elapsed between the time at which the drug was injected and the time at which its concentration was measured, as well as on the actual relationship between the drug concentration and its actual effects on the patient for the active molecule(s) of the drug at stake.

For many pharmacological laboratories or drug dosing platforms, it is common practice to provide antibiotics dosing results without any interpretation. In order to adapt dosage, the physician usually applies rules of thumbs, such as rules of three based on time-constrained measurements of drug concentrations in the patient's body. Such a method is not only prone to errors but is also limited in terms of scope since interpretation may be provided only in very few specific cases.

For instance, the physician may interpret dosing results if the drug concentration is measured one or several days after starting the treatment and at a fixed time with regard to the time at which the drug was injected into the patient and if the efficiency target of the treating molecule is defined for a peak concentration or before the next drug injection.

In those specific cases, the interpretation performed by the physician consists in manually evaluating the current dosage but does not allow determining an optimal dosing regimen for the patient in order to optimize his/her treatment.

For instance, if a concentration of amikacine is too high before injection, it is advised to make another concentration measurement later (*i.e.* after injecting the dose into the patient's body). However, this method does not allow to estimate the reinjection time (*i.e.* time at which the next dose of drug should be injected).

This represents a loss of opportunity for the patient who can seldom benefit from a drug dosing adaptation and even if such an adaptation is performed, it is not optimal for the reasons as stated above. Under these conditions, it takes time for the patient to obtain efficient drug concentrations, which increases the risk for the patient to develop either resistance to anti-infectious treatments or toxicity issues.

In order to overcome at least some of the above limitations and drawbacks, mathematical tools exploiting Bayesian models have already been proposed to estimate, from the drug concentrations measured in the patient's body, the drug concentration time-course in the patient's body regardless of the drug treatment start time and the drug concentration measurement time with regard to the drug injection time and to simulate an optimal dosing regimen knowing the target drug concentration to be reached.

For instance, such solutions are implemented in existing softwares, such as *DoseMe, BestDose* or *Ezequiel.* However, using such softwares requires that the user has performed a literature survey beforehand together with some pharmacological expertise in order to determine the target concentration. Besides, the user must simulate himself/herself the various dosage schemes or dosing regimens that may be used to reach the target concentration.

Therefore, there is a need to evaluate the dose administered to the patient in terms of efficacy and toxicity and to determine quickly, automatically and with enhanced accuracy, optimal dosing regimens of a drug that a physician may choose to administer to a patient in order to provide a treatment that is well adapted to the patient thereby overcoming the above-mentioned drawbacks.

### 3. Present invention

The present invention has been devised to address one or more of the foregoing concerns.

According to a first aspect of the invention there is provided a method for determining an optimum drug dosing regimen to treat a patient efficiently against at least one drug-sensitive pathogenic agent.

This method comprises the following steps: obtaining patient-related data; obtaining a concentration of said drug measured in the patient's body at an arbitrary time Cₘ(t); from said measured concentration Cₘ(t), computing an estimated drug concentration time-course by using a Bayesian model configured according to an initial dose previously administered to the patient, mean values of parameters selected from said patient-related data and interindividual variability of said parameters .

For example, a biological parameter of a patient such as his/her weight or his/her renal function characterizing a medical condition (*e.g.* renal failure) may be used to configure the Bayesian model. The mean value and the variance with regard to the population of treated patients may be used for that purpose. By way of example, the variance or the standard variation may used to characterize interindividual variability amongst a population of patients.

More information on how to configure the Bayesian model may be found in the following article: Fuchs A., Csajka C., Thoma Y., Buclin T., Widmer N. - Benchmarking therapeutic drug monitoring software : a review of available computer tools, Clin. Pharmacokinet. 2013 January, 52(1) : 9-22.

The initial dose is a reference dose that has been previously administered to the patient, e.g. the latest administered dose. The value of the initial dose may have been determined by implementing the method according to the present invention. The value of this initial dose may be stored in a memory accessible by a software or application as described below.

The method further comprises the following steps :
- from said estimated drug concentration time-course, determining an efficiency pharmacokinetic parameter adapted to said drug and to said patient;
- a first comparison wherein said efficiency pharmacokinetic parameter PPE is compared to at least an efficiency target CE;
- a second comparison wherein a residual drug concentration Cᵣ is obtained from said estimated drug concentration time-course and compared to at least a toxic concentration Cₜₒₓ defined as a drug concentration beyond which serious side effects occur;
- determining at least one concentration of said drug to treat said patient based on the result of said first and second comparisons to provide at least one proposed dose for said optimum dosing regimen.

According to another feature of the present invention, the efficiency target CE is defined as the product of a target ratio RC and a susceptibility factor SUS relative to said pathogenic agent, wherein:
- the target ratio RC is defined as the ratio to be reached to improve the drug efficiency based on known relationships between drug concentration and efficiency ;
- the susceptibility factor SUS is selected amongst the group of parameters including the minimal inhibiting concentration MIC, the inhibiting concentration that annihilates 90% (MIC₉₀) of the sensitive pathogenic agent, or the Epidemiological Cut Off Values ECOFF.

According to another feature of the present invention, the pathogenic agents are bacteria and the susceptibility factor (SUS) is set to be equal to:
- a maximum Epidemiological Cut-off Value (ECOFFmax) adapted to combat all bacteria that are usually sensitive and for which an ECOFF value is known or
- an ECOFFcut for an empirical treatment adapted to combat most pathogenic agents excluding species having the highest ECOFF values according to known minimal inhibiting concentration distributions.

For instance, ECOFFmax and the ECOFFcut values can be obtained from online databases.

According to another feature of the present invention, the efficiency pharmacokinetic parameter PPE is further compared to reported concentrations Cᵣₐₚ for said drug.

According to another feature of the present invention, the residual drug concentration Cᵣ is compared to a limit concentration Cₗᵢₘ defined as the drug concentration for which an increased risk of side effects is reported.

According to another feature of the present invention, the efficiency pharmacokinetic parameter PPE is greater or equal to the efficiency target, the drug concentration is considered to be sufficient.

According to another feature of the present invention, if the efficiency pharmacokinetic parameter PPE is strictly less than the efficiency target CE, a value of a percentage of drug-sensitive pathogenic agents which can be annihilated by the measured drug concentration is computed.

According to another feature of the present invention, the efficiency pharmacokinetic parameter PPE for an empirical treatment is strictly less than the efficiency target CE of certain sensitive strains of pathogenic agents, a value of a percentage of drug-sensitive pathogenic agents which can be annihilated by the measured drug concentration of drug is computed for groups of suspected pathogenic agents.

According to another feature of the present invention, the patient-related data include the following parameters:
- the group consisting of biological characteristics such as age, gender, weight, creatinin clearance, and/or,
- the group consisting of previously administered dosage such as dose, time interval between two consecutive doses, infusion duration, and/or
- the group consisting of time delay between drug administration and measurement, measured drug concentration.

According to another feature of the present invention, a set of pharmacological safety rules is applied after determining said at least one dose of said drug.

Accordingly, the method according to the present invention may further comprise a control step subsequently to providing said at least one proposed dose, wherein :
- said proposed dose is increased by not more than 50% of said initial dose, except if the proposed dose is lower than a maximal dose recommended for said drug ; and/or
- said proposed dose is decreased by not more than 50% of said initial dose, except if the proposed dose is greater than said maximal recommended dose.

The control step is advantageous to adjust (*i.e.* increase or decrease) the proposed dose, in particular when very low or very high doses have been previously administered to the patient, while keeping the proposed dose within satety limits (*i.e.* below the maximal recommended dose).

For instance, the maximal dose recommended for each drug used for treating the patients is obtained in product information or in guideline dosages for clinical practice published in the literature.

According to another feature of the present invention, the optimum dose regimen is obtained as a result of modifying the proposed dose first, then modifying a dose interval and finally modifying a duration of infusion for time-dependent antibiotics. For other categories of antibiotics (*i.e.* concentration dependent, time-and-concentration dependent), only the dose and the dose interval may be modified.

Another object of the present invention is a system comprising an application server and a client terminal, wherein the said client terminal or the application server is adapted to implement partly or fully the method as described above.

According to a feature of the present invention, the system further comprises a patient database from which the client terminal and/or the application server is adapted to retrieve patient-related data.

According to another feature of the present invention, the system further comprises a third-party database from which the client terminal and/or the application server is adapted to retrieve information on pathogenic agents and/or information on drug interactions.

At least parts of the method according to the invention as described above may be implemented by a computer or the like. Accordingly, the present invention may take the form of an entirely hardware embodiment, an entirely software embodiment (including firmware, resident software, micro-code, *etc*) or an embodiment combining both software and hardware aspects that may all generally be referred to herein as a *"circuit, "module"* or *"system".*

Furthermore, the present invention may take the form of a computer programme product embodied in any tangible medium of expression having computer usable programme code embodied in the medium.

Since the present invention can be implemented in software, the present invention can be embodied as computer readable code for provision to a programmable apparatus on any suitable carrier medium.

Thus, another object of the present invention is a computer programme comprising instructions adapted to implement any step of the method as described above when the programme is executed on a computer.

A tangible carrier medium may comprise a storage medium, such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g. a microwave or radiofrequency signal.

Thus, another object of the present invention is a medium for storing information, removable or not, readable by a computer or a microprocessor and comprising code instructions of a computer programme adapted for implementing any step of the method as described above, when the programme is executed by a computer.

### 4. Brief description of drawings

The invention will now be described according to a particular embodiment of the present invention, by way of example only and with reference to the following drawings, in which:
- **Figure 1** illustrates schematically an example of a system wherein the present invention is implemented;
- **Figure 2** is schematic block diagram of a computing device for implementing the method according to the invention;
- **Figure 3** illustrates estimated drug concentrations over time; and
- **Figure 4** illustrates the method of the invention according to a particular embodiment.

### 5. Detailed description

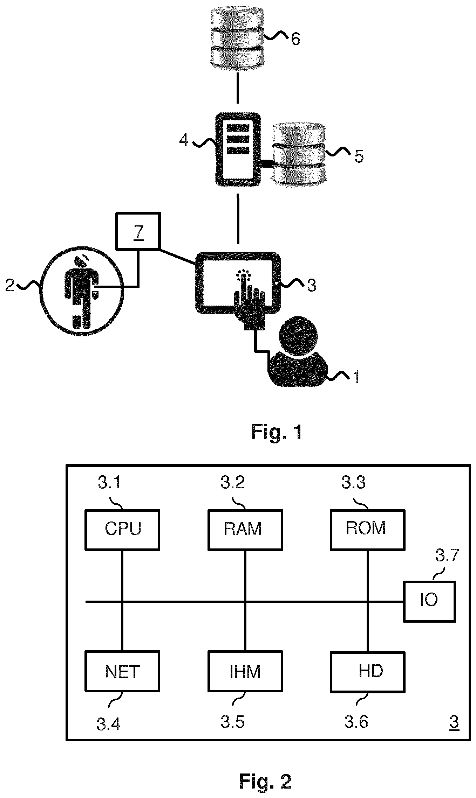

**Figure 1** illustrates schematically a system wherein the present invention may be implemented. For instance, the invention may be implemented in the context of a hospital where a physician or doctor 1 needs to decide on a dosing regimen of drug or medicine to be administered to a patient 2 infected by a pathogenic agent, such as a germ, bacteria or virus.

This system comprises a terminal 3 used by the doctor 1, an application server 4, a patient database 5 and third-party databases 6. The patient database 5 comprises information relating to patients who are being taken care of at the hospital.

The third-party databases 6 may comprise reference information relating to drugs (*e.g.* antibiotics) and pathogenic agents (*e.g.* bacteria). For instance, the third-party databases 6 may comprise one or more databases storing information on bacterial susceptibility, such as the one managed by the *European Committee on Antimicrobial Susceptibility Testing* (EUCAST), and/or databases storing information on drug interactions such as Micromedex.

The application server 4 is adapted to retrieve data from both databases 5, 6 via communication networks, such as the Internet (not illustrated). The application server 4 is also adapted to communicate with the terminal 3 via communication networks, such as the Internet and/or an intranet (not illustrated).

The system may further comprise a drug dosing device 7 adapted to measure the drug concentration in the patient's body (e.g. similarly to transdermal devices used to measure glycaemia in real-time). The drug dosing device 7 may comprise a wireless communication interface, so as to transmit and/or receive data to/from the terminal 3 via a local area network (not illustrated). The drug dosing device 7 may further be adapted to administer a dose of drug to the patient according to an instruction received from the terminal 3 operated by the doctor 1.

**Figure 2** is a schematic block-diagram of the terminal 3 used for implementing one or more embodiments of the present invention. The terminal 3 may be a laptop, tablet, smartphone or the like adapted to store and execute a programme, software or application, wherein at least part of the method of the present invention may be implemented. For example, the terminal 3 comprises a communication bus connected to:
- a central processing unit (CPU) 3.1, such as a microprocessor;
- a random access memory (RAM) 3.2 adapted to store the executable code for implementing the method of the present invention, as well as registries adapted to save data, variables and parameters necessary for implementing the method according to one or more embodiments of the invention;
- a read only memory (ROM) 3.3 for storing computer programmes, softwares or applications in order to implement the embodiments of the present invention;
- a network interface 3.4 connected to a communication network (intranet, extranet) through which medical data relating to patients, drugs, and/or bacteria may be received and/or transmitted. The network interface 3.4 may be a unique network interface or may comprise a set of different network interfaces (*e.g.* wired or wireless). Data packets are sent through the network interface or received by the network interface under the control of the application, software or programme executed by the processor 3.4;
- a user interface 3.5 for receiving user inputs and/or displaying relevant information to the user *(e.g.* displaying one or more drug doses information to the physician 1);
- an optional storage medium 3.6, such as a hard-drive or memory card (HD);
- an input/output 3.7 for receiving/sending data from/to external peripherals such as a hard-drive, removable storage media or the like.

The executable code may be stored in the read only memory 3.3, in the storage medium 3.6 or on a removable digital medium, such as a memory card.

Alternately, the executable code of the programme, software or application may be received in particular from the application server 4, by means of a communication network, through the network interface 3.4, so as to be stored in one of the storage means of the terminal 3, such as the storage medium 3.6, before being executed.

The central processing unit 3.1 is adapted to command and manage the execution of instructions or code portions of the software, application or programme according to one of the embodiments of the invention, such instructions being stored in one of the above-mentioned storage means.

After start-up, the CPU 3.1 is able to execute instructions stored in the main RAM 3.2, with regard to the software, application or programme, after these instructions have been loaded into the ROM for instance.

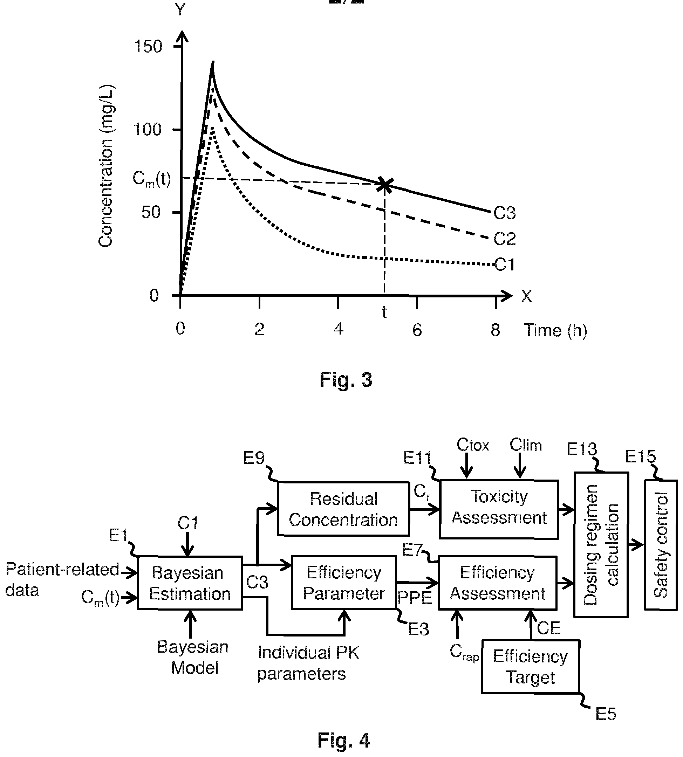

Such a software, application or programme, when it is executed by the processor 3.1, implements at least some of the steps of the diagram shown in figure 4.

In the preferred embodiment described herein, the terminal 3 is a programmable device that uses a software application configured to implement the method according to the present invention. As an alternative, the present invention may be implemented in hardware, for instance, in the form of a specific integrated circuit or *Application Specific Integrated Circuit* (ASIC).

According to the present embodiment described in reference to figures 1 and 2 above, the programme configured to implement the method of the present invention is hosted and run by the terminal 3.

In alternative embodiments, this programme may be hosted and run by the application server 4 or any remote server that has access to the patient database 5 and/or the third-party databases 6. In that case, the terminal 3 may be reduced to a lightweight client that is adapted to connect to the application server or the remote server via a communication network such as the Internet.

The present invention will now be described in the context of treating a patient against a bacteria. However, the invention is not limited to this particular framework but can also apply to any other pathogenic agents such as viruses or fungi.

For treating a patient against a bacteria, the drug or medicine considered to treat the patient is an antibiotic. It is known that antibiotics may be classified in three main categories or classes depending on whether their effect is time-dependent, concentration-dependent or concentration-and-time-dependent.

Time-dependent antibiotics (*e.g.* beta-lactams) are characterized in that the intensity of their activity on the bacteria is correlated with the duration during which the drug concentration in the patient's body exceeds a given threshold known as the *Minimum Inhibitory Concentration* (MIC).

Concentration-dependent antibiotics (*e.g.* aminosides) are characterized in that the intensity of their activity on the bacteria increases with drug concentration. Thus, a maximum or peak drug concentration must be reached in order to obtain an optimal effect on the bacteria.

Concentration-and-time-dependent antibiotics (*e.g.* fluoroquinolones) are characterized in that the intensity of their activity increases with the area under the drug concentration curve as a function of time. The area under the curve (AUC) is known as the exposure.

By default, the term *concentration* designates the total concentration of a drug in a patient's blood, which is the sum of the concentration of drug molecules in free form (*i.e.* unbound) and the concentration of drug molecules bound to the plasma proteins. This total concentration is either measured in the patient's body by sensors or estimated by means of mathematical tools.

Patient-related data may be inputted by the physician by means of the application through a human-machine interface displayed on the terminal 3. Alternatively, the patient-related data may be retrieved by the application from the patient database 5 via the application server 4 if this data is already stored in this database.

When launching the application, the physician is prompted to input, for instance through the human-machine interface by using the input/output interface 3.7, the patient's unique identifier to access his/her profile. Based on the information already available, the application may prompt the physician to complete and/or correct at least part of the patient-related data comprised within said profile.

Patient-related data may include at least one parameter selected from the group consisting of biological characteristics such as age, gender, weight, creatinin clearance or any covariable having an influence on the drug used to treat the patient. Covariable may include any treatment that may be used in combination with the administration of said drug (*i.e.* co-treatment) and that may have an impact on the pharmocokinetics of said drug, for instance by interacting with said drug.

These parameters may be advantageously integrated in a pharmacokinetic model as described below. The patient-related data may also include the following parameters selected from:
- the group consisting of previously administered dosage such as dose, time interval between two consecutive doses, infusion duration, and/or
- the group consisting of time delay between drug administration and measurement, measured drug concentration.

The patient-related data may also include parameters from the group consisting of the identification of the pathogenic agent (e.g. bacteria, virus) and the identification of the minimum inhibitory concentration of the identified pathogenic agent.

Additional information may be added to the patient's profile, such as the infection location(s) in the patient's body.

Preferably, the application is adapted to prompt the physician to input only the data which are specific to the patient to be treated, *i.e.* patient-related data, while general information concerning antibiotics and bacteria or germs have been previously stored in the application. For that purpose, the application is adapted to update the general information from third-parties databases 6 by connecting to the application server 4 via its communication interface 3.4.

In an alternate embodiment wherein the application is executed directly on the application server 4, the application is adapted to send a data update request to the terminal 3, so that the user 1 can input patient-related data to be sent to the application server 4. This embodiment is particularly advantageous to strengthen patient's data privacy, in that his/her personal data are not stored directly on user terminal 3 but on a remote server that can be secured.

There exist population pharmacokinetic models of patients allowing to describe the concentration of a drug as a function of time for an average subject. A series of reference pharmacokinetic models may be stored on the application server 4.

These models may be accessible online and retrieved by the terminal 3 upon request or may be initially stored in a memory 3.3 (ROM) of the terminal upon installation of the application.

It is assumed that such a model (PopPK) is known for the active drug molecule used to treat the patient. According to the present embodiment, this model is stored in the ROM 3.3 of the terminal 3.

According to this pharmacokinetic model, the drug concentration expressed in mg/L on the Y-axis as a function of time in hours (h) on the X-axis is shown by the doted curve C1 as illustrated schematically in **Figure 3****.**

The application is configured to adapt this pharmacokinetic model by taking into account relevant patient-related data including biological information (*e.g.* age, weight, *etc*) and clinical data, medical condition or infection status (*e.g.* renal failure). For instance, if the patient is affected by renal failure, the pharmacokinetic model is adapted not only according to his/her weight but also according to his/her renal function, so as to obtain the corrected dashed curve C2 as shown in Figure 3.

It is assumed that the drug concentration is measured in the patient's blood at an arbitrary time t. A significant advantage of the method according to the invention is that it may be applied regardless of the time at which the drug concentration is measured in the patient's blood. It results therefrom that a drug dosing regimen may be determined with more flexibility, e.g. without having to observe specific times for taking measurements as conventionally required by existing dosing techniques.

The application advantageously obtains a measured value of the drug concentration, for instance from the measuring device 7 and adapts automatically the patient's pharmacokinetic model according to this measured value.

This measurement may be performed by the measuring device 7 itself which is coupled to the terminal 3. In that case, the application is advantageously configured to receive automatically at least one measured value for the drug concentration as depicted by Cm(t) in Figure 3.

More precisely, the application is configured to estimate the drug concentration over time by applying a known Bayesian model based on said measured value. This Bayesian model is advantageously configured so as to take into account inter-individual variability of parameters, for instance selected from patient-related data, as well as mean values of these parameters. This Bayesian model is preferably stored initially with the application on the terminal 3 or may be stored in a memory of the application server 4 and/or retrieved upon request from the application server 4.

The result of this Bayesian estimation is shown in Figure 3 by the solid curve C3 that represents the estimated drug concentration over time. This curve C3 corresponds to a mathematical equation which depends on pharmacokinetic parameters for absorption, distribution and/or elimination relating to the patient (*i.e.* individual pharmacokinetic parameters). These individual pharmacokinetic parameters are obtained by Bayesian estimation.

The application is particularly advantageous in that it is adapted to compute a reliable estimation of the drug concentration automatically based on the pharmacokinetic profile expected for the patient, while taking into account his/her medical conditions (*e.g.* renal function) and a measured concentration Cₘ(t) in the patient's blood taken at an arbitrary time t. This computation is designated by step E1 in **Figure 4****.**

If an applicable pharmacokinetic model exists, it can be applied to the patient, whatever the delay between drug administration and drug measurement is. In that case, the application is adapted to estimate a concentration interval with a given prediction level that takes into account the uncertainty on said delay.

This interval is known as the *prediction interval.* For instance, with a prediction level set to 80%, the estimated concentration interval, also called the *80% prediction interval* on concentrations, is defined as the interval in which a future observation will fall, with a probability of 80%, given what has already been observed. The prediction level may be set to other values, such as 85%, 90% or 95%.

One or more patient-related parameters may also be set with a prediction interval or uncertainty when configuring the Bayesian model by means of the patient-related data.

If no pharmacokinetic model is applicable to the active molecule of the drug or to the characteristics of the patient, the application may be configured to perform efficiency analysis and toxicity analysis according to paragraphs a) and b) below if the measured concentration (sample) is a residual concentration for time-dependent antibiotics and if peak and residual concentrations are measured for concentration-dependent antibiotics.

For the remaining part of the description, it is assumed that a suitable pharmacokinetic model exists and is applicable.

### a) Efficiency analysis

Based on the individual pharmacokinetic parameters derived from the above-described Bayesian estimation, the application calculates an efficiency pharmacokinetic parameter PPE, as depicted by step E3 in Figure 4. In general terms, the efficiency pharmacokinetic parameter is defined as the pharmacokinetic measurable quantity that is most appropriately linked to the efficiency of the acting molecule of the drug used to treat the patient.

According to the present embodiment, the application is configured to define the efficiency pharmacokinetic parameter PPE according to the category of antibiotics used to treat the patient and is further configured to calculate the PPE value based on the results of the Bayesian estimation as previously obtained in step E1.

For time-dependent antibiotics, the application is configured to define the efficiency pharmacokinetic parameter as the total residual drug concentration Cᵣ in the patient's body, just before next administration of the drug to the patient.

For concentration-dependent antibiotics, the application is configured to define the efficiency pharmacokinetic parameter PPE as the maximum or peak concentration Cₚₑₐₖ.

For time-and-concentration-dependent antibiotics, the application is configured to define the efficiency pharmacokinetic parameter PPE as the exposure which corresponds to the area under the curve AUC of the drug concentration. In that case, the value of the efficiency pharmacokinetic parameter is obtained by calculating the area between the estimated curve C3 and the time axis (X-axis).

Once the efficiency pharmacokinetic parameter PPE is computed by taking into account the antibiotic category in step E3 as described above, the application is adapted to compute an efficiency target CE as shown in step E5 to which the efficiency pharmacokinetic parameter PPE is then compared in step E7.

In step E5, the application is configured to define the efficiency target CE as the product of a target ratio RC by a susceptibility factor SUS, *i.e.* CE=RCxSUS. The target ratio RC is defined namely according to the antibiotics category as described below. In general terms, the value of RC may be established from existing relationships linking drug concentration and efficiency as known in the literature which are prone to evolve as knowledge progresses. In that regard, the application may be configured to gather up-to-date data from the literature.

The susceptibility factor SUS represents the sensitivity of the bacteria to the antibiotics. The susceptibility factor SUS is defined in the application according to whether the bacteria has been identified or not, and if identified whether related parameters are known according to the different cases described below.

If the bacteria has been identified in the patient's body, the application is configured to set the susceptibility factor SUS as the *Minimum Inhibitory Concentration* (MIC) associated to the identified bacteria.

If the bacteria has been identified but its inhibiting concentrations, *e.g.* MIC, are unknown, the application is configured to set the susceptibility factor SUS equal to the *Epidemiological Cutt-off Value* (ECOFF) which corresponds to the highest minimal inhibiting concentration for any bacteria which has become sensible according to existing antibiograms. Such antibiograms may be obtained from the data retrieved in existing databases for instance managed by EUCAST. ECOFF values are determined from existing MIC distribution histograms as available on the EUCAST online databases.

If the bacteria has not been identified, the application is configured to apply a probabilistic treatment. In that case, the application is configured to define the susceptibility factor SUS as the maximum *Epidemiological Cut-off Value* (ECOFFₘₐₓ) adapted to combat all bacteria that are usually sensitive and for which an ECOFF value can be found in the EUCAST online databases.

In specific cases, the application may be further configured to define the susceptibility factor SUS as a reduced *Epidemiological Cut-Off Value* (ECOFF_{cut}) that is adapted to combat most bacteria excluding species having the highest ECOFF values according to the MIC distributions as available on the EUCAST online databases. In any case, the ECOFF_{cut} value is always lower than the ECOFFₘₐₓ value.

For concentration-dependent antibiotics such as amikacine, gentamicine, the application is configured to set the susceptibility factor SUS equal to the inhibitory concentration adapted to annihilate 90% of the sensitive bacteria (MICgo).

The application is configured to select the susceptibility factor from the parameters MIC, MlC₉₀, ECOFF, ECOFFₘₐₓ, ECOFF_{cut} as described above. This list of parameters proves to be sufficient to account for the vast majority of situations that a physician may be encounter when treating patients. These parameters may be obtained from EUCAST online databases 6 and stored locally on the terminal 3 or may be retrieved on-the-fly from the application server 4 to which the application is connected. In a first comparison step E7, the application is configured to compare the efficiency pharmacokinetic parameter PPE to the efficiency target CE=RCxSUS.

For time-dependent antibiotics, PPE is set to the total residual concentration Cᵣ that is either measured or estimated as described above in reference to Figures 3 and 4. In that case, the application is configured to compare this total residual concentration Cᵣ with CE=RCT×SUS, wherein RCT is an efficiency ratio that designates the total target ratio that the total residual concentration must reach to improve the efficiency of the active molecule of the drug. For instance, the application is configured to calculate the total target ratio RCT=Cᵣ/F, wherein Cᵣ is total residual concentration and F is the percentage of active molecules in free from *(i.e.* unbound).

For concentration-dependent antibiotics, PPE is set to the peak concentration Cₚₑₐₖ that is either measured or estimated as described above in reference to Figures 3 and 4. The application is configured to compare the peak concentration Cₚₑₐₖ with CE=RCFxSUS, wherein RCF is an efficiency ratio that designates a fixed efficiency ratio. For instance, the application is adapted to calculate RCF=PPE/MlC₉₀. By way of example, for amikacine, CE is set between 60 and 80 mg/L, which corresponds to a RCF equal to 8 to 10 times and the SUS is the highest MIC₉₀ value for the bacteria that are targeted.

For time-and-concentration dependent antibiotics, PPE is set to AUC that is calculated from the concentration curve C3 as described above in reference to Figure 3.

To sum up, the application is configured to compute the parameters as shown in Table 1 below and determine in the first comparison step E7 whether the condition PPE ≥ CE is verified.

**Table 1. Efficiency parameters computed by application**

| **Antibiotics Category** | **PPE** | **CE = RC × SUS** | |
|---|---|---|---|
| Time-dependent | Cᵣ | RC=RCT | SUS = MIC, ECOFF or ECOFFmax, ECOFFcut |
| Concentration-dependent | Cₚₑₐₖ | RC=RCF | SUS = MIC₉₀ |
| Time & concentration-dependent | AUC | RC | SUS = MIC, ECOFF or ECOFFmax, ECOFFcut |

In general terms, if the PPE ≥ CE=RCxSUS, the drug concentration is considered to be satisfactory.

Otherwise, the application may be configured to determine and display on the terminal 3 relevant information that is valuable to the physician for administering a drug to the patient, such as an estimation of the percentage of bacteria that can be combatted by the measured drug concentration Cm(t). Indeed, the physician may react differently in a situation where only 20% of the bacteria can be fought and in a situation where as much as 95% of the bacteria can be fought.

If PPE < CE = RC × SUS, the application may be adapted to display:
- the value *"insufficient concentration or exposition (AUC)"* if the MIC of the bacteria is known;
- the value of a *"satisfactory concentration or exposition for a certain percentage of sensitive strains of the bacteria",* if the bacteria is known but its MIC is unknown;
- the value of a *"satisfactory concentration or exposition for a certain percentage of the sensitive strains of a first bacteria and another percentage of the sensitive strains of a second bacteria"* in case of probabilistic treatment.

In step E7, the efficiency pharmacokinetic parameter PPE or the efficiency target CE=RC×SUS may be compared to reference drug concentrations that are reported in the literature Crap. Indeed, if the SUS is much lower than standard values (e.g. CE < 10×Cᵣₐₚ), the PPE will also be compared to reported concentrations in the literature, for time-dependent or time-and-concentration-dependent antibiotics. If the efficiency pharmacokinetic parameter is much lower than reference drug concentration as reported in the literature (*e.g.* PPE < 10×Cᵣₐₚ), a dose adaptation could be proposed depending on the clinical context.

### b) Toxicity Analysis

In step E9, the application determines the residual concentration Cᵣ which is defined as the total concentration of a drug that remains in the patient's body just before the next administration of the drug. Step E9 may be automatically executed in parallel to determining the efficiency parameter in step E3, as soon as Bayesian estimation is performed in step E1.

It is understood that this total residual concentration Cᵣ is the sum of the concentration of drug molecules in free form (*i.e.* unbound) and the concentration of drug molecules bound to the plasma proteins. The total residual concentration is either measured in the patient's body by sensors (*e.g.* by means of a measuring device 7) or estimated by the application from the estimated concentration evolution computed in step E1 (*i.e.* corresponding to curve C3 as depicted on Figure 3).

In a second comparison step E11, the application compares the residual drug Cᵣ concentration to a toxic concentration Cₜₒₓ defined as a drug concentration beyond which serious side effects occur. This comparison occurs only when the residual drug Cᵣ concentration is higher than the reported concentrations Cᵣₐₚ.

In some cases, the application may be configured to further compare the residual drug concentration Cᵣ to a limit concentration Cₗᵢₘ defined as the drug concentration for which an increased risk of side effects is reported, for instance in online databases to which the application has access. This concentration may be overpassed, if it is considered that the benefits of the treatment are higher than its risks. The application is configured to perform this additional comparison, when the residual concentration is higher than reported concentrations and lower than the toxic concentration.

For time-dependent antibiotics, the application is configured to determine first whether the residual concentration Cᵣ is greater than the toxic concentration Cₜₒₓ (*i.e*. Cᵣ > Cₜₒₓ) and then whether the residual concentration Cᵣ is greater than the limit concentration Cₗᵢₘ (*i.e*. Cᵣ > Cₗᵢₘ).

For concentration-dependent antibiotics, the application is configured to determine whether the residual concentration Cᵣ is greater than the toxic concentration Cₜₒₓ. Limit concentrations are actually not determined for this class of antibiotics.

For time-and-concentration-dependent antibiotics, the application is configured to determine whether the residual concentration Cᵣ is greater than the limit concentration Cₗᵢₘ. The limit concentration may be replaced by the highest concentration reported in the literature without reported adverse events.

Based on the results of the first and second comparison steps E7, E11, the application is configured to generate and display a message on the terminal to provide appropriate recommendations or warnings to the physician 1. In particular, the application is configured to determine if the residual concentration Cᵣ is greater than the limit concentration Cₗᵢₘ and/or if the residual concentration Cᵣ is greater than the toxic concentration Cₜₒₓ, *i.e.* Cᵣ > Cₗᵢₘ and/or Cᵣ > Cₜₒₓ and the application is adapted to display this information.

For instance, the application may indicate whether the residual concentration is satisfactory, insufficient or toxic together with background information relating to the *concentration-effect* relationships which have been referred to for setting the limit and/or toxic concentrations.

### c) Dose calculation

Based on the results of the first and second comparison steps E7, E11, the application is further configured to compute in step E13 a dosing regimen by calculating at least one of the following parameters: a dose, dose interval, an infusion duration. This calculation is performed by the application by taking into account the antibiotic category (*i.e.* time-dependent, concentration-dependent, time-and-concentration dependent).

The application is configured to run simulations by adjusting at least one of these three parameters and provide optimum dosing regimens.

For time-dependent antibiotics, the application is configured to determine the following parameters:
- a target dose, *i.e.* a dose to administer to the patient so that the peak concentration corresponds to the middle of the target interval (*e.g.* target being between 8 and 10 times the MIC₉₀);
- a time interval between two consecutive doses ;
- a time for re-injection (*i.e.* for injecting another dose) defined as the time before the drug concentration becomes lower than a predetermined threshold (*e.g.* C < 0.8 × Ctox).

For any other antibiotics, the application is adapted to calculate one or more of the following dosing regimens (*i.e.* dose, dose interval, infusion duration) which are adapted to reach the efficiency target while meeting the toxicity constraints as defined above:
- DoseMIC: defined as a dose, dose interval, infusion duration to administer so that PPE/MIC=RC;
- DoseECOFF: defined as a dose, dose interval, infusion duration to administer so that PPE/ECOFF=RC;
- DoseECOFFₘₐₓ: defined as a dose, dose interval, infusion duration to administer so that PPE/ECOFFₘₐₓ=RC;
- DoseECOFF_{cut}: defined as a dose, dose interval, infusion duration to administer so that PPE/ECOFF_{cut}=RC;
- DoseRap: defined as a dose, dose interval, infusion duration to administer so that the residual concentration reaches reported concentrations Crap;
- Doselim: defined as a dose, dose interval, infusion duration to administer so that the residual concentration is equal to the limit concentration Cₗᵢₘ;
- Dosetox: defined as a dose, dose interval, infusion duration to administer so that the residual concentration reaches the toxic concentration Cₜₒₓ but without overrunning Cₜₒₓ.

Dose calculations are made by the application using the equation of the concentration as a function of time, described in the literature, with the individual parameters calculated for the patient. A first dose and dosing interval modification are proposed by the application. For instance, for time-dependent concentrations, if this modification is not sufficient, an increase of the duration of infusion is proposed by the application.

In particular embodiments, the application is adapted to propose several doses. For illustration purposes, we consider the example of an empirical treatment supposing that the concentration is insufficient to fight against all the sensitive strains of the bacteria but already above the limit concentration. In that case, the application is adapted to propose a first dose equal to DoseECOFFmax to fight against 100% of the sensitive strains and a second dose equal to Doselim to reach the highest concentration, thereby limiting toxicity risks. Thus, the application allows the clinician to adapt the dose as a function of the patient's clinical context, *e.g.* either increase the dose to DoseECOFFmax for lack of efficacy or decrease the dose to Doselim in case of adverse events.

Existing safety rules may be applied after calculating the doses to adjust the proposed dose within safety limits. For that purpose, the application is adapted to apply safety rules in a control step E15 after providing the dose at the outcome of step E13. These safety rules may be predefined in the application and updated or retrieved from existing reference databases.

During the control step E15, the proposed dose may be increased or decreased as a function of the initial dose and the recommended maximal dose. By default, the initial dose is defined in the application as the most recent dose that was administered to the patient. The history of all administered doses is stored by the application.

The application is adapted to read the value of the initial dose that was stored in a memory of the application server or in the memory of the terminal from/on which the application is executed.

For instance, the proposed dose is increased by not more than 50% of said initial dose, except if the proposed dose is lower than the maximal recommended dose.

For instance, the proposed dose is decreased by not more than 50% of said initial dose, except if the proposed dose is greater than said maximal recommended dose.

For time-and-concentration dependent antibiotics, if the exposure is lower than CE, a DoseMIC or DoseECOFF is determined, residual concentration produced is then compared to the limit concentration.

For all drugs, a maximum 50% increased dose will be proposed, except if this new dose remains under the maximal recommended dose. A maximum 50% decreased will be proposed except if the new dose remains greater than the maximal recommended dose or for important over-dosage.

Although the present invention has been described hereinabove with reference to a specific embodiment, the present invention is not limited to this specific embodiment and modifications will be apparent to a person skilled in the art which as falling within the scope of the present invention.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

### List of abbreviations

- AUC: Area under curve
- CE: Efficiency Target
- Cₗᵢₘ: Limit Concentration
- Cₘ: Measured Concentration
- Cᵣ: Residual Concentration
- Cᵣₐₚ: Reported Concentration
- Cₜₒₓ: Toxic Concentration
- ECOFF: Epidemiological cut-off value
- EUCAST: European Committee on Antimicrobial Susceptibility Testing
- MIC: Minimum inhibitory concentration
- PPE: Efficiency Pharmacokinetic Efficiency
- SUS: Susceptibility

## Claims

1. Method for determining an optimum drug dosing regimen to treat a patient (2) efficiently against at least one drug-sensitive pathogenic agent, said method comprising the following steps:
- obtaining patient-related data;
- obtaining a concentration of said drug measured in the patient's body at an arbitrary time (Cₘ(t));
- from said measured concentration (Cₘ(t)), computing (E1) an estimated drug concentration (C3) time-course by using a Bayesian model configured according to :
• an initial dose previously administered to the patient;
• mean values of parameters selected from said patient-related data
• interindividual variability of said parameters ;
said method being **characterized in that** it further comprises :
- from said estimated drug concentration time-course (C3), determining an efficiency pharmacokinetic parameter (PPE) adapted to said drug and to said patient;
- a first comparison (E7) wherein said efficiency pharmacokinetic parameter (PPE) is compared to at least an efficiency target (CE);
- a second comparison (E11) wherein a residual drug concentration (Cᵣ) is obtained from said estimated drug concentration time-course (C3) and compared to at least a toxic concentration (Cₜₒₓ) defined as a drug concentration beyond which serious side effects occur;
- determining (E13) at least one concentration of said drug to treat said patient based on the result of said first and second comparisons to provide at least one proposed dose for said optimum dosing regimen.

2. Method according claim 1, **characterized in that** the efficiency target (CE) is defined as the product of a target ratio (RC) and a susceptibility factor (SUS) relative to said pathogenic agent, wherein :
- the target ratio (RC) is defined as the ratio to be reached to improve the drug efficiency based on known relationships between drug concentration and efficiency ;
- the susceptibility factor (SUS) is selected amongst the group of parameters including the minimal inhibiting concentration (MIC), the inhibiting concentration that annihilates 90% (MIC₉₀) of the sensitive pathogenic agent, or the Epidemiological Cut Off Values (ECOFF).

3. Method according to claim 2, **characterized in that** pathogenic agents are bacteria and the susceptibility factor (SUS) is set to be equal to:
- a maximum Epidemiological Cut-off Value (ECOFFmax) adapted to combat all bacteria that are usually sensitive and for which an ECOFF value is known or
- an ECOFFcut for an empirical treatment adapted to combat most pathogenic agents excluding species having the highest ECOFF values according to known minimal inhibiting concentration distributions.

4. Method according to any of the preceding claims, **characterized in that** the efficiency pharmacokinetic parameter (PPE) is further compared to reported concentrations (Cᵣₐₚ) for said drug.

5. Method according to any of the preceding claims, **characterized in that** the residual drug concentration (Cᵣ) is compared to a limit concentration (Cₗᵢₘ) defined as the drug concentration for which an increased risk of side effects is reported.

6. Method according to claim 2, **characterized in that** if the efficiency pharmacokinetic parameter (PPE) is greater or equal to the efficiency target, the drug concentration is considered to be sufficient.

7. Method according to claim 2, **characterized in that**, if the efficiency pharmacokinetic parameter (PPE) is strictly less than the efficiency target (CE), a value of a percentage of drug-sensitive pathogenic agents which can be annihilated by the measured drug concentration is computed.

8. Method according to claim 2, **characterized in that**, if the efficiency pharmacokinetic parameter (PPE) for an empirical treatment is strictly less than the efficiency target (CE) of certain sensitive strains of pathogenic agents, a value of a percentage of drug-sensitive pathogenic agents which can be annihilated by the measured drug concentration of drug is computed for groups of suspected pathogenic agents.

9. Method according to any of the preceding claims, **characterized in that** it further comprises a control step (E15) subsequently to providing said at least one proposed dose (E13), wherein:
- said proposed dose is increased by not more than 50% of said initial dose, except if the proposed dose is lower than a maximal dose recommended for said drug ; and/or
- said proposed dose is decreased by not more than 50% of said initial dose, except if the proposed dose is greater than said maximal recommended dose.

10. Method according to any preceding claims, **characterized in** said optimum dose regimen is obtained as a result of modifying the proposed dose first, then modifying a dose interval and finally modifying a duration of infusion for time-dependent antibiotics.

11. Method according to any one of claims 1 to 9, **characterized in that** said optimum dose regimen is obtained as a result of modifying the proposed dose and the dose interval for concentration-dependent antibiotics or for time and concentration-dependent antibiotics.

12. System comprising an application server (4) and a client terminal (3), **characterized in that** said client terminal (3) or said application server (4) is adapted to implement the method according to any one of claims 1 to 11.

13. System according to claim 12, **characterized in that** it further comprises a patient database (5) from which the client terminal (3) and/or the application server (4) is adapted to retrieve patient-related data.

14. System according to claim 12 or 13, **characterized in that** it further comprises a third-party database (6) from which the client terminal (3) and/or the application server (4) is adapted to retrieve information on pathogenic agents and/or information on drug interactions.

15. Computer programme comprising instructions adapted to implement any step of the method according to any one of claims 1 to 11 when the programme is executed on a computer (3).

16. Medium for storing information, removable or not, readable by a computer (3) or a microprocessor (3.1) and comprising code instructions of a computer programme adapted for implementing any step of the method according to any one of claims 1 to 11, when said programme is executed by a computer.
